# EUROPÄISCHE PATENTSCHRIFT

(11) **EP 0 291 784 B1**
(45) Veröffentlichungstag und Bekanntmachung des Hinweises auf die Patenterteilung: **17.03.1993**
(21) Anmeldenummer: 88107305.0
(22) Anmeldetag: 06.05.1988
(51) Int. Cl.: A61B 5/02

(54) **Vorrichtung zur Vasometrie**
Device for vascular measurements
Appareil destiné à des mesures vasculaires

(30) Priorität: 21.05.1987 DE 3717046
(43) Veröffentlichungstag der Anmeldung: 23.11.1988
(73) Patentinhaber: B.V. Optische Industrie "De Oude Delft", 2600 MD Delft (NL)
(72) Erfinder: Affeldt, Karl-Heinz, Dipl.-Ing., D-1000 Berlin 19 (DE); Hantel, Ulrich, Dipl.-Ing., D-1000 Berlin 65 (DE)

(56) Entgegenhaltungen:
- FR-A- 2 296 396
- R. VOELKER: "Herz- und Gefässerkrankungen", Kreislauf-Buecherei, Band 16, Seiten 152-157, 1957, D. Steinkopff Verlag, Darmstadt, DE

## Beschreibung

Die Erfindung betrifft eine Vorrichtung nach dem Oberbegriff des Anspruchs 1.

### Stand der Technik

Es ist bekannt (Prof. Völker, Herz- und Gefäßerkrankungen, Kreislaufbücherei, Band 16, Darmstadt, 1957), Durchblutungsstörungen mittels Vasographie festzustellen. Zu diesem Zweck wird zum Beispiel an einem Zeh des Fußes eines Probanden ein Durchlicht- oder Reflexionslichtsensor angebracht, beispielsweise mittels eines Klettenbandes. Die mit dem Sensor durch Abtastung der Volumenpulse erhaltenen elektrischen Signale werden nach einer Verstärkung auf einem Sichtgerät als Kurvenverlauf sichtbar gemacht und gegebenenfalls mit einer Schreibvorrichtung aufgezeichnet. Durch einen Vergleich der an einem Zeh des linken Fußes und an einem Zeh des rechten Fußes erhaltenen Kurvenverläufe kann der Fachmann unter anderem Schlüsse auf den Zustand der Blutgefäße ziehen. Ein wesentlicher Nachteil der bekannten Meßmethode besteht darin, daß das Meßergebnis von der Art der Befestigung des Sensors an dem Zeh sowie vom Ruhighalten des Fußes des Probanden während der Messung abhängt.

### Aufgabe

Der Erfindung liegt die Aufgabe zugrunde, eine Vorrichtung anzugeben, die sicherstellt, daß der Proband während der Messung seinen Fuß möglichst locker und entspannt in einer fixierten Lage hält und daß der Sensor mit einer nur geringen mechanischen Vorspannung an dem Zeh anliegt, damit das durch die Druckwelle des Blutes erzeugte Ausdehnen des Adern in dem Zeh so wenig wie möglich behindert wird.

### Lösung und erzielbare Vorteile

Diese Aufgabe wird bei einer gattungsgemäßen Vorrichtung durch die kennzeichnenden Merkmale des Anspruchs 1 gelöst.

Die mit der Erfindung erzielten Vorteile bestehen insbesondere darin, daß der Proband in die Lage versetzt wird, seinen Fuß völlig entspannt derart auf der Vorrichtung ruhen zu lassen, daß mit dem Sensor in Berührung stehende Zeh während der Messung seine Lage unverändert beibehält und daß der Sensor durch seine federnde Lagerung nur mit einer sehr geringen Kraft auf den Zeh drückt. Um eine an den Probanden optimal angepaßte Andruckkraft des Volumenpulssensors zu erhalten, ist es nach einer weiteren Ausbildung der Erfindung vorteilhaft, wenn der Volumenpulssensor derart federnd gelagert ist, daß die Federkraft einstellbar ist.

### Beschreibung eines Ausführungsbeispiels

Ein Ausführungsbeispiel der Erfindung ist in der Zeichnung anhand dreier Figuren dargestellt und wird in der Zeichnung näher beschrieben. Es zeigen
- Fig. 1: eine Ansicht der erfindungsgemäßen Vorrichtung,
- Fig. 2: eine Seitenansicht zu Fig. 1 und
- Fig. 3: eine nichtmaßstäbliche Schnittansicht entsprechend dem Schnittverlauf III in Fig. 2.

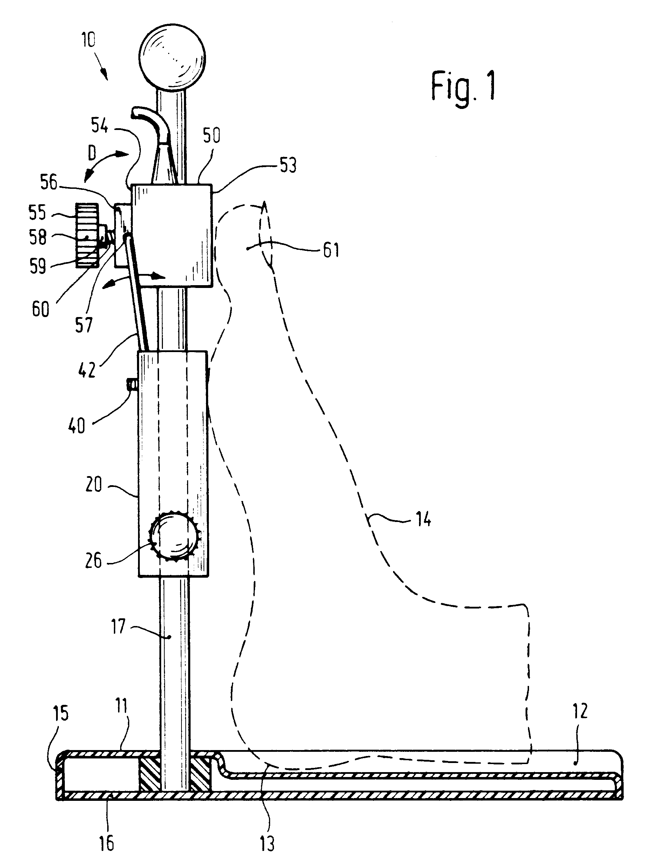

In den Fig. 1 bis 3 ist 10 eine Vorrichtung zur Vasometrie, die eine horizontale Grundplatte 11 mit einer länglichen, muldenförmigen Vertiefung 12 zur Aufnahme einer Ferse 13 eines menschlichen Fußes 14 aufweist. Die Grundplatte besteht aus einer flachen Gehäuseschale 15 und einer fest damit verbundenen, die offene Seite der Gehäuseschale abdeckenden Bodenplatte 16. In der Grundplatte 11 sind zwei vertikale Säulen 17, 18 befestigt, auf denen eine Traverse 20 vertikal verschiebbar angeordnet ist. Die Traverse umfaßt ein wannenförmiges, längliches Gehäuse 21, auf dessen Bodenwand 22 zwei Lagerklötze 23, 24 mit dem Durchmesser der Säulen 17, 18 entsprechenden Lagerbohrungen 25 befestigt sind.

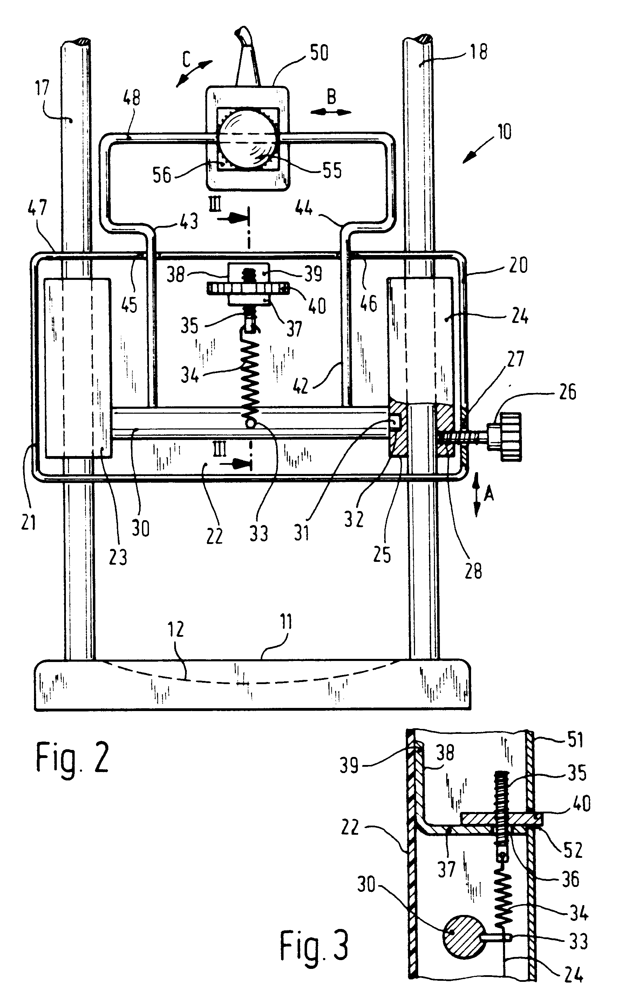

Eine Arretierschraube 26, die durch eine Öffnung 27 des wannenförmigen Gehäuses 21 hindurchgesteckt und in ein senkrecht zur Lagerbohrung 25 angeordnetes Gewindeloch 28 des Lagerklotzes 24 eingeschraubt ist, dient zum Arretieren der Traverse 20 in einer bestimmten Höhe über der Grundplatte 11. Innerhalb der Traverse 20 befindet sich zwischen den beiden Lagerklötzen 23 und 24 eine horizontale Welle 30, deren im Durchmesser abgesetzte Enden 31 in entsprechende Bohrungen 32 der Lagerklötze 23, 24 passen, so daß die Welle drehbar ist. Vorzugsweise auf halber Länge der Helle 30 trägt diese einen radialen Stift 33, der mit einem Ende eines Federelementes 34, das ist vorzugsweise eine Zugfeder, verbunden ist. Das andere Ende des Federelementes ist mit einem Ende eines Gewindebolzens 35 verbunden, der in eine Öffnung 36 (Fig. 3) eines horizontalen Schenkels 37 eines Winkelstücks 38 gesteckt ist. Oberhalb des horizontalen Schenkels 37 ist auf den Gewindebolzen 35 eine Mutter 40 geschraubt, die als Rändelmutter ausgebildet ist. Ein vertikaler Schenkel 39 des Winkelstücks ist an der Bodenwand 22 des wannenförmigen Gehäuses 21 befestigt.

Etwa senkrecht zu dem radialen Stift 33 ist in der Welle 30 und radial zu dieser ein U-förmiger Bügel 42, das ist vorzugsweise ein Drahtbügel, befestigt, dessen Schenkel 43, 44 durch längliche Öffnungen 45, 46 der oberen Stirnseite 47 des wannenförmigen Gehäuses 21 nach außen ragen. Ein horizontaler Abschnitt 48 des Bügels 42 trägt einen Volumenpulssensor 50, das ist vorzugsweise ein Reflexionslichtsensor.

Die offene Seite des wannenförmigen Gehäuses 21 schließt eine Platte 51 (Fig. 3) ab, die eine längliche Öffnung 52 zum freien Durchtritt eines Teils der Mutter 40 aufweist.

Der vorzugsweise quaderförmige Volumenpulssensor ist folgendermaßen mit dem horizontalen Abschnitt 48 des Bügels 42 verbunden. In die Rückseite 54 des Volumenpulssensors 50 ist eine zweite Arretierschraube 55 eingeschraubt, die eine Druckplatte 56 durchsetzt, welche eine Nut 57 zur Aufnahme des horizontalen Abschnitts 48 des Bügels enthält. Die zweite Arretierschraube 55 besteht ebenso wie die erste Arretierschraube 26 aus einem runden Knopf 58, einem koaxial damit verbundenen zylindrischen Ansatz 59 und einem mit diesem verbundenen Gewindebolzen 60.

Die Funktion der vorstehend beschriebenen Vorrichtung 10 ist folgende.

Unter die Ferse 13 des vorzugsweise auf einer Liege liegenden Probanden wird die Vorrichtung 10 geschoben, so das die Ferse 13 auf der Vertiefung 12 der Grundplatte 11 ruht. Dann wird zunächst nach dem Lösen der ersten Arretierschraube 26 die Traverse 20 derart in der Höhe verstellt (Pfeil A in Fig. 2), daß die Mitte des Zehs 61 des Fußes 14 mit der Mitte der Frontseite 53 des Volumenpulssensors 50 übereinstimmt. Danach wird die erste Arretierschraube 26 fest angezogen. In der Folge wird die zweite Arretierschraube 55 nur lose angezogen und der Volumenpulssensor 50 in horizontaler Richtung verschoben (Pfeilrichtung B in Fig. 2), gegebenenfalls um seine horizontale Achse (Pfeilrichtung C in Fig. 2) und um den horizontalen Abschnitt 48 (Pfeilrichtung D in Fig. 1) gedreht und damit auf den Zeh 61 optimal ausgerichtet. Abschließend wird die zweite Arretierschraube 55 fest angezogen. Durch das Anziehen der zweiten Arretierschraube 55 drückt deren Ansatz 59 auf die Druckplatte 56, die den in ihrer Nut 57 liegenden horizontalen Abschnitt 48 des Bügels 42 fest gegen die Rückseite 54 des Volumenpulssensors 50 preßt, so daß der Sensor in der eingestellten Lager unverrückbar festgehalten wird. Die auf den Volumenpulssensor 50 ausgeübte Federkraft der Feder 34 (Fig. 2) wird durch Drehen der Mutter 40 (Fig. 3) eingestellt, wobei der Gewindebolzen 35 nach oben bzw. nach unten wandert. Nachdem die Einstellung der Federkraft beendet ist, kann die eigentliche Messung beginnen.

Die Säulen 17 und 18, der Bügel 42 und die Welle 30 bestehen vorzugsweise aus einem nichtrosenden Stahl und die Gehäuseschale 15 sowie das Gehäuse 21 aus einem wärmeisolierenden Kunststoff.

## Patentansprüche

1. Vorrichtung zur Vasometrie (10) im Bereich der Zehen des menschlichen Fußes mit einem Volumenpulssensor (50), **dadurch gekennzeichnet,** daß die Vorrichtung (10) eine Grundplatte (11) mit einer Vertiefung (12) zur Aufnahme der Ferse (13) des Fußes (14) sowie auf der Grundplatte (11) angeordnete Haltemittel (17) und eine daran befestigte Traverse (20) aufweist, wobei der Volumenpulssensor (50) federnd gelagert an der Traverse (20) befestigt ist.

2. Vorrichtung nach Anspruch 1, dadurch gekennzeichnet, daß der Volumenpulssensor (50) an der Vorrichtung (10) bzw. an der Traverse (20) derart befestigt ist, daß seine horizontale und vertikale Lage, der Winkel zwischen seiner Längsachse und der Ebene der Grundplatte (11) sowie der Winkel zwischen der Querachse des Volumenpulssensors und der Ebene der Grundplatte einstellbar und arretierbar sind.

3. Vorrichtung nach Anspruch 1 oder 2, dadurch gekennzeichnet, daß die Haltemittel aus zwei senkrecht auf der Grundplatte (11) befestigten Säulen (17, 18) bestehen, auf der die Traverse (20) vertikal verschiebbar und arretierbar angeordnet ist.

4. Vorrichtung nach Anspruch 3, dadurch gekennzeichnet, daß die Traverse (20) einen um eine Achse parallel zur Längsache der Traverse schwenkbaren U-förmigen Bügel (42) aufweist, auf dessen horizontalen Abschnitt (48) der Volumenpulssensor (50) längsverschiebbar, um den horizontalen Abschnitt schwenkbar, um seine Längsachse drehbar und arretierbar angebracht ist.

5. Vorrichtung nach Anspruch 4, dadurch gekennzeichnet, daß der Bügel (42) entgegen der Kraft eines Federelementes (34) um eine horizontale Achse schwenkbar ist, die innerhalb der Traverse (20) liegt.

6. Vorrichtung nach Anspruch 4 oder 5, dadurch gekennzeichnet, daß die durch die Längsverschiebung des Volumenpulssensors (50) auf dem horizontalen Abschnitt (48) des Bügels (42), durch die Drehung um die Längsachse des horizontalen Abschnitts (48) und die durch Drehung um die Längsachse des Volumenpulssensors eingestellte räumliche Lage des Volumenpulssensors mittels einer zweiten Arretierschraube (55) arretierbar ist, die mit ihrem Gewindebolzen (60) durch eine Druckplatte (56) hindurch in die Rückseite (54) des quaderförmigen Volumenpulssensors eingeschraubt ist und die Druckplatte, die in ihrer Nut (57) den horizontalen Abschnitt (48) des Bügels (42) aufnimmt, gegen die Rückseite des Volumenpulssensors preßt.

7. Vorrichtung nach Anspruch 5, dadurch gekennzeichnet, daß der U-förmige Bügel (42) mit den Enden seiner Schenkel (43, 44) diametral an einer Welle (30) befestigt ist, die innerhalb der Traverse (20) drehbar gelagert ist und die horizontale Schwenkachse für den Bügel bildet.

8. Haltevorrichtung nach Anspruch 7, dadurch gekennzeichnet, daß ein radialer Stift (33) der Welle (30) über ein Federelement (34) mit einem Einstellelement (40) zum Einstellen der Federkraft verbunden ist.

9. Haltevorrichtung nach Anspruch 8, dadurch gekennzeichnet, daß das Federelement eine Zugfeder (34) ist.

10. Vorrichtung nach Anspruch 1 oder einem der folgenden, dadurch gekennzeichnet, daß der Volumenpulssensor (50) ein Reflexionslichtsensor ist.

11. Vorrichtung nach Anspruch 1, dadurch gekennzeichnet, daß die Grundplatte (11) aus einer länglichen, flachen Gehäuseschale (15) und einer die offene Seite der Gehäuseschale abdeckenden Bodenplatte (16) besteht.

12. Vorrichtung nach Anspruch 1, 3, 4, 7 oder 8, dadurch gekennzeichnet, daß die Traverse (20) aus einem wannenförmigen Gehäuse (21) und einer die offene Seite des Gehäuses abdeckenden Platte (51) besteht und zwei Lagerklötze (23, 24) mit Lagerbohrungen (25) für die Säulen (17, 18), die in den Lagerklötzen drehbar gelagerte Welle (30), die an der Welle befestigten Schenkel (43, 44) des Bügels (42), das Federelement (34) sowie das damit verbundene Einstellelement (40) enthält.

## Claims

1. Apparatus (10) for vascular measurement in the region of the toes of the human foot using a volume pulse sensor (50), characterized in that the apparatus (10) has a base plate (11) with a depression (12) for receiving the heel (13) of the foot (14) and retaining means (17) located on the base plate (11) and a crossbeam (20) feed thereto, in which the volume pulse sensor (50) is feed in resiliently mounted manner to the crossbeam (20).

2. Apparatus according to claim 1, characterized in that the volume pulse sensor (50) is feed to the apparatus (10) or to the crossbeam (20) in such a way that its horizontal and vertical position, the angle between its longitudinal His and the plane of the base plate (11), as well as the angle between the transverse axis of the volume pulse sensor and the plane of the base plate are adjustable and lockable.

3. Apparatus according to claims 1 or 2, characterized in that the retaining means comprise two columns (17, 18) vertically feed to the base plate (11) and on which the crossbeam (20) is arranged in vertically displaceable and lockable manner.

4. Apparatus according to claim 3, characterized in that the crossbeam (20) has a U-shaped clip (42) pivotable about an axis parallel to the longitudinal axis of the crossbeam and to whose horizontal portion (48) the volume pulse sensor (50) is fitted in longitudinally displaceable manner, so as to pivot about the horizontal portion, rotate about its longitudinal axis and so as to be lockable.

5. Apparatus according to claim 4, characterized in that the clip (42) can be pivoted about a horizontal axis located within the crossbeam (20) in opposition to the tension of a spring element (34).

6. Apparatus according to claims 4 or 5, characterized in that the spatial position of the volume pulse sensor set through the longitudinal displacement of said sensor (50) on the horizontal portion (48) of the clip (42), by rotation about the longitudinal axis of the horizontal portion (48) and by the rotation about the longitudinal axis of said sensor can be locked by means of a second locking screw (55), whose threaded bolt (60) is screwed through a pressure plate (56) into the back (54) of the parallelepipedic volume pulse sensor and presses the pressure plate, which receives in its groove (57) the horizontal portion (48) of the clip (42), against the back of the volume pulse sensor.

7. Apparatus according to claim 5, characterized in that the U-shaped clip (42) is feed by the ends of its legs (43, 44) diametrically to a shaft (30), which is mounted in rotary manner within the crossbeam (20) and forms the horizontal pivot axis for the clip.

8. Retaining apparatus according to claim 7, characterized in that a radial pin (33) of the shaft (30) is connected by means of a spring element (34) to a setting member (40) for setting the spring tension.

9. Retaining apparatus according to claim 8, characterized in that the spring element is a tension spring (34).

10. Apparatus according to claim 1 or one of the following claims, characterized in that the volume pulse sensor (50) is a reflected light sensor.

11. Apparatus according to claim 1, characterized in that the base plate (11) comprises an elongated, flat casing shell (15) and a base plate (16) covering the open side of the casing shell.

12. Apparatus according to claims 1, 3, 4, 7 or 8, characterized in that the crossbeam (20) comprises a trough-shaped casing (21) and a plate (51) covering the open side of the casing and contains two bearing blocks (23, 24) with bearing bores (25) for the columns (17, 18), the shaft (30) mounted in rotary manner in the bearing blocks, the legs (43, 44) of the clip (42) feed to the shaft, the spring element (34) and the setting element (40) connected thereto.

## Revendications

1. Appareil pour des mesures vasculaires (10) dans le domaine des orteils du pied humain au moyen d'un capteur volumétrique d'impulsions (50), caractérisé en ce que l'appareil (10) présente un socle (11) muni d'un renfoncement (12) pour recevoir le talon (13) du pied (14) ainsi que des moyens de maintien (17) disposés sur le socle (11) et une traverse (20) fixée sur ces derniers, le capteur volumétrique d'impulsions (50) étant positionné sur la traverse (20) par une liaison élastique,

2. Appareil selon la revendication 1, caractérisé en ce que le capteur volumétrique d'impulsions (50) est fixé à l'appareil (10), respectivement à la traverse (20), de telle sorte que sa position horizontale et verticale, l'angle entre son axe longitudinal et le plan du socle (11) ainsi que l'angle entre l'axe transversal du capteur volumétrique d'impulsions et le plan du socle puissent être réglés et bloqués.

3. Appareil selon la revendication 1 ou 2, caractérisé en ce que les moyens de maintien consistent en deux colonnes (17, 18) fixées verticalement sur le socle (11) sur lesquelles la traverse (20) est disposée de manière à pouvoir coulisser verticalement et être bloquée,

4. Appareil selon la revendication 3, caractérisé en ce que la traverse (20) présente un étrier (42) en forme de U qui peut pivoter autour d'un axe parallèlement à l'axe longitudinal de la traverse et sur la portion horizontale (48) duquel le capteur volumétrique d'impulsions (50) étant disposé de manière à pouvoir coulisser dans le sens de la longueur, pivoter autour de la portion horizontale et pivoter et être bloqué autour de son axe longitudinal.

5. Appareil selon la revendication 4, caractérisé en ce que l'étrier (42) est placé pour pouvoir pivoter autour d'un axe horizontal, disposé à l'intérieur de la traverse (20), en s'opposant à la farce d'un élément élastique (34).

6. Appareil selon la revendication 4 ou 5, caractérisé en ce que la position dans l'espace du capteur volumétrique d'impulsions, qui est réglée au moyen du coulissement du capteur volumétrique d'impulsions (50) dans le sens de la longueur sur la portion horizontale (48) de l'étrier (42), de la rotation autour de l'axe longitudinal de la portion horizontale (48) et de la rotation autour de l'axe longitudinal du capteur volumétrique d'impulsions, peut être bloquée au moyen d'une deuxième vis de blocage (55) qui, au moyen de sa tige filetée (60), est vissée dans la face arrière (54) du capteur volumétrique d'impulsions parallélépipédique en traversant une plaque de serrage (56), et que la plaque de serrage qui reçoit dans sa rainure (57) la portion horizontale (48) de l'étrier (42) s'appuie sur la face arrière du capteur volumétrique d'impulsions.

7. Appareil selon la revendication 5, caractérisé en ce que l'étrier en forme de U (42) est fixé diamétralement par les extrémités de ses côtés (43, 44) à un arbre (30) qui est positionné à rotation à l'intérieur de la traverse (20) et constitue pour l'étrier l'axe horizontal de pivotement.

8. Dispositif de fixation selon la revendication 7, caractérisé en ce qu'une tige radiale (33) de l'arbre (30) est reliée par l'intermédiaire d'un élément élastique (34) à un élément de réglage (40) pour régler la force de l'élément élastique.

9. Dispositif de fixation selon la revendication 8, caractérisé en ce que l'élément élastique (34) est un ressort de traction.

10. Appareil selon la revendication 1 ou une des revendications suivantes, caractérisé en ce que le capteur volumétrique d'impulsions (50) est un capteur de lumière réfléchie.

11. Appareil selon la revendication 1, caractérisé en ce que le socle (11) consiste en un bâti allongé en forme de coque (15) et en une plaque de fond (16) recouvrant le côté ouvert du bâti en forme de coque.

12. Appareil selon la revendication 1, 3, 4, 7 ou 8, caractérisé en ce que la traverse (20) consiste en un boîtier en forme de cuve (21) et une plaque (51) obturant le côté ouvert du boîtier et comprend deux blocs de fixation (23, 24) munis de trous de passage (25) pour les colonnes (17, 18), l'arbre (30) situé à rotation dans les blocs de fixation, les côtés (43, 44) de l'étrier (42) fixes à l'arbre, l'élément élastique (34) de même que l'élément de réglage (40) qui lui est relié.
